# EUROPEAN PATENT APPLICATION

(11) **EP 3 348 259 A1**
(43) Date of publication of application: **18.07.2018**
(21) Application number: 16844628.4
(22) Date of filing: 01.09.2016
(51) Int. Cl.: A61K 9/16, A61K 47/30, A61K 47/38, A61K 47/42, A61K 9/00, C08J 3/12

(54) **POLYMER MICROSPHERE HAVING SPONTANEOUS PORE-CLOSURE FUNCTIONALITY AND METHOD FOR PREPARING SAME**

(30) Priority: 11.09.2015 KR 20150129183
(71) Applicant: Ewha University-Industry Collaboration Foundation, Seoul 03760 (KR)
(72) Inventor: SAH, Hong-kee, Seoul 03626 (KR)
(74) Representative: Agasse, Stéphane
(86) International application number: PCT/KR2016/009797
(87) International publication number: WO 2017/043808

(57) **Abstract**

The present invention relates to a method for preparing a polymeric microsphere and the polymeric microsphere prepared by the method, and specifically, relates to a method for preparing a polymeric microsphere in which a single oil-in-water type of emulsion is prepared using specific polymeric compound and organic solvent and then ammonia solution is added to perform ammonolysis and the polymeric microsphere prepared thereby, a composition for bioactive substance delivery comprising the microsphere and a method for preparing thereof.
The preparation method of the present invention does not use a halogen organic solvent which is harmful to human body, and does not use a porogen and uses ammonolysis, and in case of preparing a polymeric microsphere according to the preparation method, a microsphere which the preparation process is simple of and is not harmful to human body and has spontaneous pore-closing functionality and extremely strong porosity and pores.

## Description

### [TECHNICAL FIELD]

The present invention relates to a polymeric microsphere and a method for preparing the same, and specifically relates to a porous microsphere with spontaneous pore-closing functionality, a method for preparing the same, and a composition for drug delivery comprising the microsphere.

### [BACKGROUND ART]

Conventional injection forms such as infusions, suspensions and emulsions are rapidly removed from the body after muscle or subcutaneous administration, so frequent injections are essential for the treatment of chronic diseases. Microencapsulation designed in order to solve this problem refers to a preparation process of encapsulating drug into a microsphere (hereinafter, the microsphere includes a nanosphere) formulation consisting of a polymeric compound, and since the microsphere can be administered by muscle or subcutaneous injection to human or animal, because it commonly has um unit of size, and it can be prepared to have various drug release rates, the drug delivery period can be controlled. Therefore, it is possible to maintain an effective therapeutic drug concentration for a long period with only one administration, thereby minimizing the total administration amount of drug required for the treatment, and enhancing the compliance of drug treatment of patients, and thus, currently, the outstanding worldwide pharmaceutical companies have shown their great interests in the preparation of polymeric microspheres containing drug.

As the preparation method of microspheres using biodegradable polymers as above, a solvent evaporation drying method (U.S. Patent No. 4,652,441), a phase separation method (U.S. Patent No. 4,675,189), a spray drying method (U.S. Patent No. 6,709,650) and a low temperature solvent extraction method (U.S. Patent No. 5,019,400), etc. are known. However, the microspheres prepared as above are mostly non-porous, and have a disadvantage that their preparation is not easy and a lot of time is taken and the release rate of encapsulated drug is slow due to non-porosity.

In order to prepare porous microspheres in which the disadvantage that the release rate of encapsulated drug is slow due to non-porosity, a double emulsion using porogens and organic solvents such as methylene chloride and ethyl acetate, specifically W1/O/W2 (water-in-oil-in-water) emulsion solvent evaporation extraction method has been used, and the W1/O/W2 emulsion solvent evaporation extraction method is a method using that porogens present in W1 are diffused into W2 and at the same time, water of W2 permeates to W1, due to large osmosis difference between W1 and W2 of W1/O/W2 emulsion, thereby finally preparing porous microspheres. Many of methods for preparing microspheres using such double emulsion as above have been reported (Int J Pharm 2007;333:103, J Controlled Release 133(2009)37, Angew Chem Int Ed 51(2012)10800, ACS Macro Lett 1(2012)697 and Ch Chem Lett 24(2013)710, etc), and all of these methods using the double emulsion use an organic solvent such as methylene chloride or ethyl acetate as a dispersion solvent, or certainly use NaCl, trehalose, BSA, peroxide, cyclodextrin, and the like.

However, methylene chloride used as the organic solvent has a big problem that it is a cancer-causing substance and causes environmental toxicity, although it is an optimized organic solvent, since it has strong volatility, is not mixed with water well, and has the boiling point further lower than water, and it requires an inconvenient process that the emulsion should be stirred overnight to remove it from the emulsion (U.S. Patent No. 6,884,435), and the temperature of reactor is raised or decompression condition is introduced to shorten the preparation time of microspheres (refer to U.S. Patent No. 3,691,090, No. 3,891,570, No. 6,270,700 and No. 6,572,894). Moreover, in addition to this inconvenience of processes, there is a problem that drug is denatured by passing the above processes. In addition, high concentration of porogens should be added to W1 to secure porosity, but there is a problem that drug dissolved in W1 is release to the external phase, W2, due to the increase of osmosis caused at that time, and furthermore, there is a problem that drug encapsulation efficiency is declined, as the drug is also removed in the step of removing porogens.

On the other hand, the present inventors have disclosed a method for preparing a polymeric microsphere comprising after preparing an emulsion by adding a dispersed phase comprising a polymeric compound, drug and a water-insoluble organic solvent into a dispersion solvent, ammonia solution is added to the prepared emulsion and reacted (Korean Registered Patent No. 910892 and U.S. Patent No. 8,202,525). However, in this preparation method, non-porous microspheres are prepared by using a high molecular weight of polymeric compound and a halogen solvent such as methyl chloroacetate, etc.

### [DISCLOSURE]

### [TECHNICAL PROBLEM]

Under these circumstances, the present inventors continued research to prepare a porous microsphere which does not have problems of encapsulation efficiency of drug and drug denaturation and can also exhibit appropriate drug release rate and can be produced environmentally friendly by a simple process. As a result, it was demonstrated that in case of selecting specific dispersion solvent and polymer, preparing single O/W emulsion by not comprising a porogen, and using it to prepare a porous microsphere in a sponge shape by ammonolysis, the microsphere can have spontaneous pore-closing functionality which means that pores are self-closed in an aqueous phase, although it has porosity, and as a result, it was confirmed that a porous polymeric microsphere which can encapsulate a biomacromolecule can be prepared simply, efficiently, and environmentally friendly.

Therefore, one purpose of the present invention is to provide a method for preparing a porous polymeric microsphere with spontaneous pore-closing functionality and a polymeric microsphere prepared according to that.

In addition, another purpose of the present invention is to provide a composition for drug delivery comprising the polymeric microsphere as an active ingredient.

### [TECHNICAL SOLUTION]

As one aspect for solving the above purposes, the present invention relates to a method for preparing a porous microsphere having spontaneous pore-closing functionality.

Specifically, in order that the porous microsphere has spontaneous pore-closing functionality, the preparation method according to the present invention is characterized by comprising the steps as follows:
(a) a step of preparing a dispersed phase by contacting or mixing a polymeric compound having 1,000 to 80,000 of weight average molecular weight and an organic solvent;
(b) a step of preparation of emulsion which prepares oil-in-water (O/W) type of emulsion by adding a dispersion solvent into the dispersed phase prepared in the step (a); and
(c) a step of ammonolysis which performs ammonolysis by adding ammonia solution into the oil-in-water type of emulsion prepared in the step (b),
wherein the organic solvent is one or more non-halogen organic solvents selected from isopropyl formate, methyl formate, ethyl formate, propyl formate and mixtures of these organic solvents and a semipolar solvent.

### [ADVANTAGEOUS EFFECTS]

When a polymeric microsphere is prepared by the method for preparing a polymeric microsphere according to the present invention, there are advantages that effective delivery of bioactive substances is possible, since not only the polymeric microsphere which delivers bioactive substances by a simple and environmentally friendly process can be prepared safely and harmlessly to human body without using a porogen and harmful organic solvent which are necessarily required in the past, but also the polymeric microsphere prepared according to that has spontaneous pore-closing functionality even though it is a porous microsphere, thereby eluting drug in the body with a proper dissolution rate and also having high encapsulation efficiency of bioactive substances.

### [DESCRIPTION OF DRAWINGS]

FIG. 1 is a drawing schematizing the mechanism of formation of pores and empty holes inside·outside of PLGA microsphere.
FIG. 2 is SEM images of microspheres prepared by fixing the amount of isopropyl formate and aqueous ammonia used to 4ml and 3ml, respectively and varying the amount of PLGA used to (a) 0.15, (b) 0.25, (c) 0.35 and (d) 0.45 g.
FIG. 3 is SEM images of surfaces of microspheres produced by preparing a dispersed phase by dissolving 0.45 g of PLGA in 4 ml of isopropyl formate and emulsifying it in an aqueous solution, and then adding dropwise 3 ml of aqueous ammonia, and the right image are the left images taken at high magnification.
FIG. 4 is SEM images of insides of microspheres produced by preparing a dispersed phase by dissolving (a) 0.15, (b) 0.25, (c) 0.35 and (d) 0.45 g of PLGA in 4 ml of isopropyl formate and emulsifying it in an aqueous solution, and then adding dropwise 3 ml of aqueous ammonia, and the right image are the left images taken at high magnification..
FIG. 5 is SEM images showing surfaces of PLGA microspheres produced by emulsifying a dispersed phase prepared by dissolving 0.35 g of PLGA in (a) 4, (b) 5, and (c) 6 ml of isopropyl formate in an aqueous solution to form emulsion, and then adding dropwise 3, 3.5, and 4.1 ml of aqueous ammonia.
FIG. 6 is SEM images showing insides of PLGA microspheres produced by emulsifying a dispersed phase prepared by dissolving 0.35 g of PLGA in (a) 4, (b) 5, and (c) 6 ml of isopropyl formate in an aqueous solution to form emulsion, and then adding dropwise 3, 3.5, and 4.1 ml of aqueous ammonia.
FIG. 7 relates to SEM images of surfaces of microspheres prepared by emulsifying a dispersed phase prepared by dissolving 0.25 g of PLGA in 4 ml of isopropyl formate in an aqueous solution to form emulsion, and then adding dropwise (a) 3, (b) 5, (c) 7, and (d) 9 ml of aqueous ammonia.
FIG. 8 relates to SEM images of insides of microspheres prepared by emulsifying a dispersed phase prepared by dissolving 0.25 g of PLGA in 4 ml of isopropyl formate in an aqueous solution to form emulsion, and then adding dropwise (a) 3, (b) 5, (c) 7, and (d) 9 ml of aqueous ammonia.
FIG. 9 is SEM images of surfaces of microspheres produced by preparing a dispersed phase by dissolving (a) 0.15, (b) 0.25, (c) 0.35 and (d) 0.45 g of PLGA (iv = 0.49 dL/g) in 4 ml of isopropyl formate and emulsifying it in an aqueous solution, and then adding dropwise 3 ml of aqueous ammonia.
FIG. 10 is SEM images of insides of microspheres produced by preparing a dispersed phase by dissolving (a) 0.15, (b) 0.25, (c) 0.35 and (d) 0.45 g of PLGA (iv = 0.49 dL/g) in 4 ml of isopropyl formate and emulsifying it in an aqueous solution, and then adding dropwise 3 ml of aqueous ammonia. The right image are the left images taken at high magnification.
FIG. 11 is SEM images showing external shapes of microspheres observed (b) before dispersion, and after (b) 0.5, (c) 1, (d) 1.5, and (e) 2 hours from the dispersion of PLGA microspheres in a 37°C aqueous solution. The right image are the left images taken at high magnification.
FIG. 12 is SEM images showing internal shapes of microspheres observed (b) before dispersion, and after (b) 0.5, (c) 1, (d) 1.5, and (e) 2 hours from the dispersion of PLGA microspheres in a 37°C aqueous solution.
FIG. 13 is SEM images showing external shapes of microspheres observed (b) before dispersion, and after (b) 0.5, (c) 1, (d) 1.5, and (e) 2 hours from the dispersion of PLGA microspheres in a 41.7°C aqueous solution. The right image are the left images taken at high magnification.
FIG. 14 is SEM images showing internal shapes of microspheres observed (b) before dispersion, and after (b) 0.5, (c) 1, (d) 1.5, and (e) 2 hours from the dispersion of PLGA microspheres in a 41.7°C aqueous solution, to evaluate the spontaneous closing functionality of sponge microspheres. The right image are the left images taken at high magnification.
FIG. 15 is drawings showing the fluorescence microscope test result demonstrating that FITC-dextran can be encapsulated with the porous PLGA microsphere prepared in advance.
FIG. 16 is drawings showing the confocal laser scanning microscope test result demonstrating that FITC-dextran can be encapsulated with the porous PLGA microsphere prepared in advance.
FIG. 17 is a schematic diagram showing the preparation of a non-porous PLGA microsphere which disperses a sponge shape of porous PLGA prepared in advance in a biomacromolecule aqueous solution, thereby encapsulating the biomacromolecule using spontaneous pore and air hole-closing functionality.
FIG. 18 is photographs observing the internal and external shapes of porous polymers with spontaneous pore-closing functionality according to the present invention prepared by using ethyl cellulose as a polymeric compound through SEM.
FIG. 19 is photographs observing the internal and external shapes of porous polymers with spontaneous pore-closing functionality according to the present invention prepared by using polycaprolactone as a polymeric compound through SEM.

### [BEST MODE]

Hereinafter the present invention will be described in more details.

In the present invention, the polymeric compound is characterized by having 1,000 to 80,000 of weight average molecular weight. Any kind of this polymeric compound known in the art can be used as long as it has the weight average mean molecular weight in the above range without limitation, biodegradable polymers as well as non-biodegradable polymers may be used. This polymer may be one or more kinds selected from the group consisting of polylactic acid, polyglycolic acid, polylactide, polylactic-co-glycolic acid, polylactide-co-glycolide, polyphosphazene, polyiminocarbonate, polyphosphoester, polyanhydride, polyorthoester, copolymer of lactic acid and caprolacton, polycaprolacton, polyhydroxyvalerate, polyhydroxybutyrate, polyamino acid, polydioxanone, copolymer of lactic acid and amino acid, cellulose esters and cellulose ethers (for example, ethyl cellulose), but not limited thereto. Preferable, as the polymer, polylactide-co-glycolide, polylactide, polycaprolactone or ethyl cellulose which has 1,000 to 80,000 of weight average molecular weight may be used.

As above, the polymer used in the preparation method according to the present invention is characterized by using the polymer having 1,000 to 80,000 of weight average molecular weight. The conventional preparation method of microsphere using ammonolysis uses a polymeric compound having high molecular weight of which molecular weight is over the above range, and thus when using the polymeric compound having high molecular weight, non-porous microspheres are produced. Specifically, if the molecular weight is too high, the viscosity of dispersed phase will be higher, and thereby ammonolysis in the dispersed phase is inhibited and at the same time it is difficult for by-products, that is water-soluble ingredients, to be diffused into a dispersion solvent effectively, and as a result, ultimately, non-porous microspheres are produced.

This polymeric compound is prepared as a dispersed phase by being contacted or mixed with an organic solvent. The organic solvent used then is a non-halogen solvent which does not comprise a halogen in the molecule, and may be isopropyl formate, methyl formate, ethyl formate, propyl formate, or mixtures of these organic solvents. In addition, in addition to the aforementioned specific non-halogen organic solvents, the organic solvent may be a mixture with a semipolar solvent as a cosolvent (cosolvent system), and the semipolar solvent may be for example, dimethyl sulfoxide, acetone, acetonitrile, tetrahydrofuran, dimethyl formamide, toluene, etc. The organic solvents used for ammonolysis in Korean Registered Patent No. 910892 and U.S. Patent No. 8,202,525, etc. in the past are all halogenated organic solvents, and they are solvents which are difficult to be practically used in the pharmaceutical industry due to its own toxicity, and only non-porous microspheres are produced by them, but in the present invention, organic solvents are non-halogenated organic solvents which have relatively less toxicity and also can prepare porous microspheres and they can be directly applied to the pharmaceutical industry.

In addition, the dispersed phase prepared by using the polymeric compound and organic solvent produces an oil-in-water (O/W) type of single emulsion by being mixed with a dispersion solvent in the step (b). The dispersion solvent used then should prepare the oil-in-water type of emulsion, it is preferable to use an aqueous dispersion solvent. As the aqueous dispersion solvent, an aqueous solution comprising a hydrophilic emulsifier, for example, polyvinyl alcohol or an emulsifier like Tween family, or its cosolvent. The concentration of emulsifier comprised in the dispersion solution may be 0.1 to 4 %(w/w), preferably 0.5 to 2.0 %(w/v).

In the step (a), the ratio of the polymeric compound and dispersion solvent for preparing the dispersed phase, may be adjusted so that the concentration of polymeric compound in the prepared dispersed phase is 3(w/v)% to 30(w/v)%, preferably 3.75(w/v)% to 15(w/v)%. In this regard, when the concentration of polymer is over 30(w/v)%, the viscosity of dispersed phase is increased, thereby inhibiting ammonolysis in the following step, step (c) and inhibiting diffusion of by-products of ammonolysis, and therefore the level of porosity formation is decreased, and when it is less than 3(w/v)%, there is a problem that the mechanical strength of microsphere is weak and therefore its practicality is decreased. In specific one example, the dispersed phase was prepared by dissolving 0.15 to 0.45 g of polymeric compounds in 4 ml of organic solvent. In addition, when the dispersed phase prepared in the step (a) is mixed with the dispersion solvent, its mixing volume ratio is 1:1 to 1:100, preferably 1:4 to 1:10. In this regard, there is a problem that when the amount of dispersion solvent is too much, before inducing ammonolysis that is before adding ammonia most of organic solvents composing the dispersed phase are diffused into an aqueous phase directly, and thereby it becomes a hardened microsphere shape, not an emulsion droplet, and then, when ammonia is added, the microsphere has not porosity, and thus a microsphere having porosity of sponge shape cannot be prepared.

In addition, in the step (c), ammonolysis is performed by adding an ammonia solution into the oil-in-water type of single emulsion prepared in the step (b), and then, the amount of ammonia solution used is adjusted based on the volume of organic solvent used in the step (a), and it is preferable that the mole ratio of organic solvent: ammonia solution is 1:0.5 to 1:20. In this regard, there may be a problem that when the trace amount of ammonia outside the range is used, pores are not formed, or when the excess amount of ammonia outside the range is used, very many of pores are formed, but decomposition of polymer is increased at the same time, thereby weakening the mechanical strength of microspheres. In specific one example, ammonolysis is progressed by using 3 to 7 mL of 28% ammonia to 4ml of isopropyl formate.

By the method for preparing the microsphere according to the present invention, different from the conventional preparation processes of porous microspheres, the dispersed phase in which the polymeric compound is dissolved in the non-halogen organic solvent like isopropyl formate is emulsified in an aqueous solution, thereby forming O/W type of emulsion, and then a chemical reaction derivative which can be diffused into emulsion droplets in the aqueous phase as ammonia and decompose the non-halogen dispersion solvent into an aqueous solvent is added to the O/W emulsion, and as a result, the non-halogen organic solvent composing emulsion droplets is decomposed into the aqueous solvent (for example, in case of isopropyl formate, isopropanol and formaldehyde, as the aqueous solvent). In these aqueous solvents, the polymeric compound is not dissolved, and therefore polymeric compound-rich domain and polymeric compound-free domain are classified in emulsion droplets, and the aqueous solvents are diffused spontaneously into the aqueous phase, and thus microspheres of polymeric compound are formed. The process occurs very rapidly, and as a result, a step of solvent extraction or solvent evaporation using large amount of quenching liquid is not required at all, and O/W type of emulsion can be easily prepared as an emulsifier of low energy, and thus, special machinery or high-energy or high-shear mixing device is not required. In particular, in case of biomacromolecules, there is a risk that biomolecules may be denatured, aggregated, or decomposed, due to a process of being exposed to the organic solvent consisting of dispersed phase or being exposed to the organic solvent/water interface, or shear by a high-energy mixing device used for emulsion formation. However, when the present porous microsphere is utilized, there is an advantage that a bioactive substance like biomacromolecules can be encapsulated safely in the 100% aqueous condition. In addition, when microspheres are prepared by the preparation method according to the present invention, there is an advantage that the significant porous structure can be secured without using porogens which are necessarily required in the conventional preparation techniques of porous microspheres.

Moreover, as another embodiment, the present invention relates to a microsphere in a sponge shape with spontaneous pore-closing functionality, prepared according to the method.

Specifically, the microsphere according to the present invention, has a unique characteristic that it has sponge shape of pores or air holes present on the surface as well as inside and they are organically connected to each other to form a network.

On the other hand, the term of "spontaneous pore-closing functionality" used herein, means that internal and external pores of microspheres are self-closed without help of other materials, thereby reducing the area of pores in contact with the surfaces of microspheres, resulting in a compact matrix form. The spontaneous pore-closing starts when 100% aqueous solution microspheres without organic solvents are dispersed, and it is affected by the surrounding temperature of microspheres, and specifically, this spontaneous pore-closing occurs around the glass transition temperature (T_{g}) of microspheres or at slightly higher temperature.

In the present invention, the polymeric microsphere may have 20 to 1,000um, preferably 50 to 350um of average diameter, but not limited thereto, and it can be freely modified according to the desired use. Then, the average diameter of microsphere may be determined by viscosity of emulsion, concentration of polymeric compound of dispersed phase, molecular amount of polymeric compound, volume ratio of dispersed phase and dispersion solvent, concentration of emulsifier, etc.

As such, the microsphere according to the present invention has an advantage that it can release drug persistently for a long period when used as a drug carrier by having a sponge shape of porosity, and it exhibits excellent encapsulation efficiency of drug due to the spontaneous pore-closing functionality.

As another embodiment, the present invention relates to a composition for bioactive substance delivery comprising the porous polymeric microsphere with spontaneous pore-closing functionality and a bioactive substance.

Specifically, the bioactive substance means all materials which can exhibit a desired effect in a living body. The bioactive substance may be biomacromolecules, for example, peptide, polypeptide, polynucleotide, protein, nanoparticle, DNA, RNA, monoclonal antibody, or vaccine, but not limited thereto. As preferable one embodiment, the composition for bioactive substance delivery according to the present invention may comprise a carrier, an excipient, an additive, etc. commonly used for a pharmaceutical composition, in addition to the porous polymeric microsphere and bioactive substance.

In addition, as another embodiment, the present invention relates to a method for preparing the composition for bioactive substance delivery. Specifically, the method for preparing the composition for bioactive substance delivery comprises the steps as follows:
a step of preparing the spontaneous pore-closing polymeric microsphere; and
a step of encapsulating a bioactive substance into the microsphere.

In preferable one embodiment, the step of encapsulating the bioactive substance may comprise a step of contacting the microsphere and the bioactive substance and a step of rising temperature. In preferable one embodiment, the step of rising temperature may be carried out by rising temperature up to the glass transition temperature of microsphere.

### [MODE FOR INVENTION]

Hereinafter, the present invention will be described in more details by the following examples. However, these examples are intended to illustrate the present invention, and the scope of the present invention is not limited by these examples.

### Example 1. Preparation of microspheres

A dispersed phase was prepared by dissolving 0.15, 0.25, 0.35 or 0.45 g of PLGA (lactide:glycolide ratio = 75:25; inherent viscosity = 0.25 dL/g in chloroform) into 4 ml of isopropyl formate. The dispersed phase was dispersed into 40 ml of 0.5 %(w/v) polyvinyl alcohol (molecular weight = 25,000 g/mol; hydrolysis level = 88%) and stirred at 450 rpm for 3 min using a magnetic stirrer to prepare an emulsion by emulsification. Then 3 ml of ammonia water (ammonia concentration 28%) was added into the emulsion and stirred for 30 min continuously. Then a microsphere suspension was prepared by adding 40 ml of water and then it was filtered through a filter paper with the 11 mm pore size to collect microspheres. The obtained microspheres were dispersed into 80 ml of 0.1% polyvinyl alcohol (PVA) again and stirred for 2 hours. After that, microspheres were obtained by filtering them through a filter paper and then dried for one night under the vacuum condition.

The shapes of dried individual microsphere were observed by SEM (Model JSM-5200; Jeol Inc.; USA). In particular, two kinds of methods were used to observe the internal shapes of microspheres. Specifically, the first method was to disperse porous microspheres in a sponge shape on a double-sided fixed in a metal stub. Then, the tape was peeled off, after pressing it by u tape sing another tape. Insides of microspheres were exposed by peeling off the surface layers of microspheres through the above process. However, insides of microspheres which have nano size of pores or have not pores were not able to be observed by the first method. That was because the surface layers of microspheres were not peeled off. In this case, as the second method, after mixing the microsphere samples with epoxy resin to harden, microspheres were cut by using a knife and their insides were exposed. Afterward, the shapes of microsphere samples were observed by using SEM.

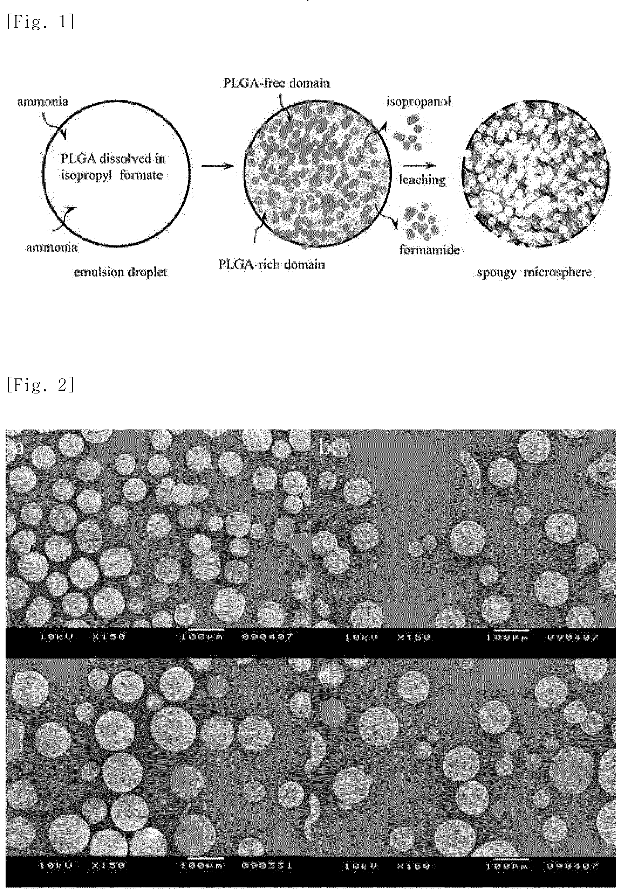

FIG. 2 shows SEM micrographs which observe microspheres prepared by varying the capacity of PLGA used for the dispersed phase from 0.15 to 0.25, 0.35 and 0.45. It showed that prepared microspheres had excellent flexibility, and this demonstrated that aggregation phenomena between microspheres during the drying process were not severe. Microspheres prepared by the present experimental conditions mostly had the size between 20 and 200 mm.

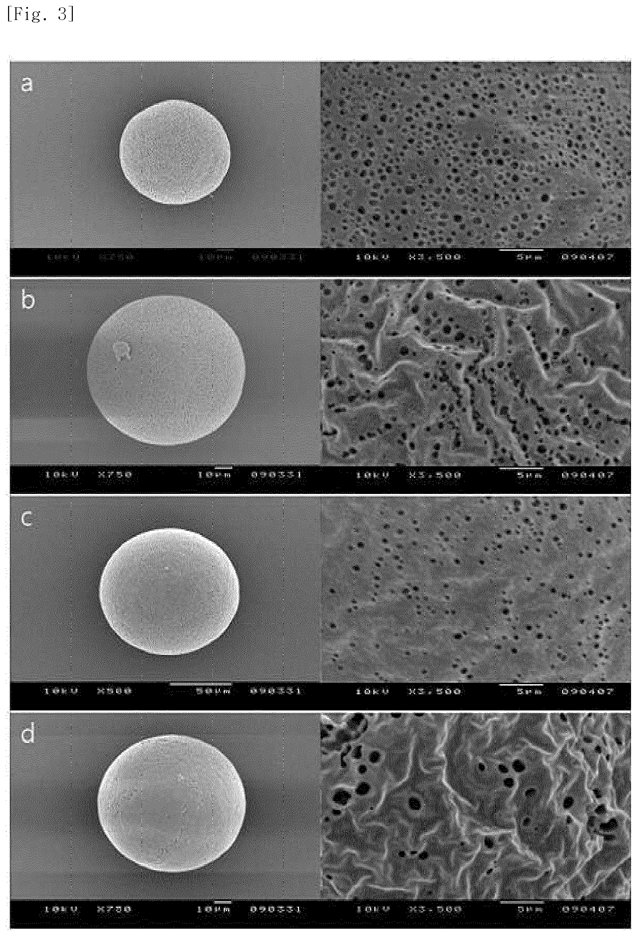

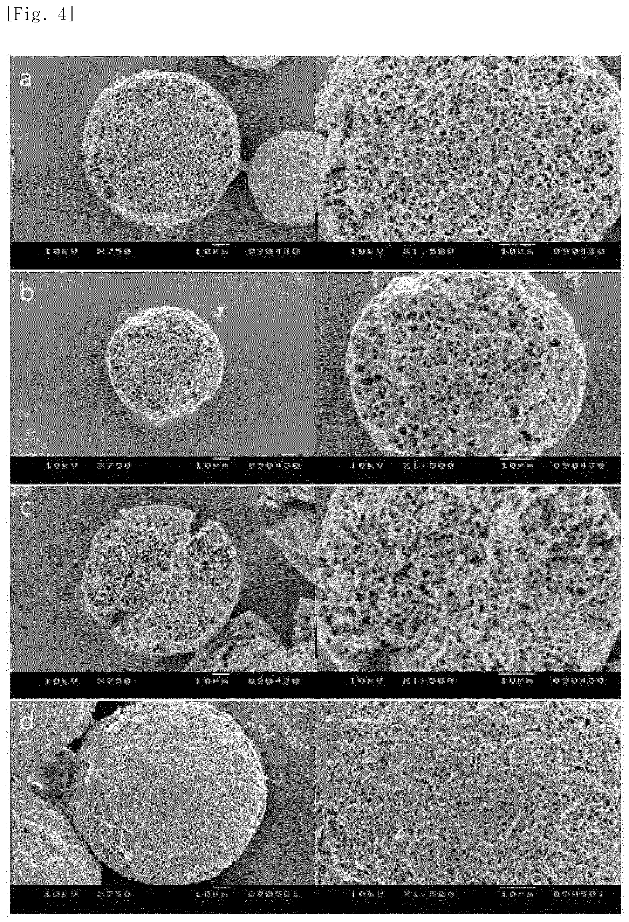

FIG. 3 is that individual microsphere shown in FIG. 2 was observed by magnification. When microspheres were prepared by using 0.15 g of PLGA, the surfaces of exhibited significant porosity. When the capacity of used PLGA was increased by 0.25, 0.35 and 0.45 g, they showed that the level of porosity and the number of air holes were relatively decreased. FIG.4 is SEM micrographs observing insides of these microspheres. It showed that the microspheres prepared by the present invention had extremely strong porosity and also had a sponge shape in which innumerable air holes were connected each other. This extremely strong porosity represents the completely differentiated figure from porous PLGAs reported in the conventional references. The more special fact is that the significant porosity was secured, although porogens which are necessarily required for the conventional preparation methods of porous microspheres were not used, and much simpler o/w emulsion method than W1/O/W2 emulsion method was used.

### Example 2. Preparation of microspheres

A dispersed phase was prepared by dissolving 0.35 g of PLGA (lactide:glycolide ratio = 75:25; inherent viscosity = 0.25 dL/g in chloroform) into 4, 5 or 6 ml of isopropyl formate. The dispersed phase was dispersed into 40 ml of 0.5 %(w/v) polyvinyl alcohol (molecular weight = 25,000 g/mol; hydrolysis level = 88%) and stirred at 450 rpm for 3 min using a magnetic stirrer to prepare an emulsion by emulsification. Then 3, 3.5 or 4.1 ml of ammonia water (ammonia concentration 28%) was added into the emulsion and stirred for 30 min continuously. Then a microsphere suspension was prepared by adding 40 ml of water and then it was filtered through a filter paper with the 11 mm pore size to collect microspheres. The obtained microspheres were dispersed into 80 ml of 0.1% polyvinyl alcohol again and stirred for 2 hours. After that, microspheres were obtained by filtering them through a filter paper and then dried for one night under the vacuum condition.

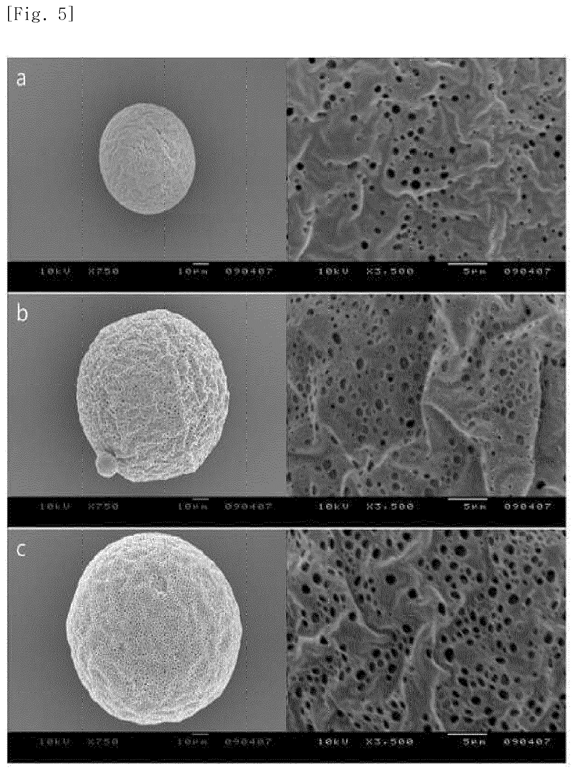

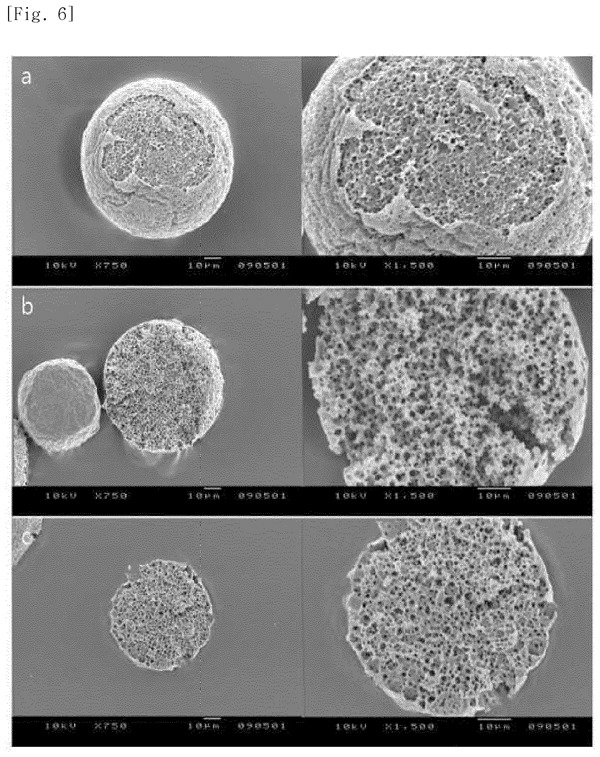

FIG. 5 showed that the porosity of microsphere surfaces was changed according to the capacity of isopropyl formate and the capacity of ammonia water used to solubilize 0.35 g of PLGA. When the capacity of isopropyl formate was increased and at the same time the capacity of ammonia water used was increased, the level of porosity of microsphere surfaces and the number of air holes tended to be increased. When comparing internal porosity after separating corresponding microsphere surface layers likewise, the same phenomena were shown (FIG. 6).

### Example 3. Preparation of microspheres

A dispersed phase was prepared by dissolving 0.25 g of PLGA (lactide:glycolide ratio = 75:25; inherent viscosity = 0.25 dL/g in chloroform) into 4 ml of isopropyl formate. The dispersed phase was dispersed into 40 ml of 0.5 %(w/v) polyvinyl alcohol (molecular weight = 25,000 g/mol; hydrolysis level = 88%) and stirred at 450 rpm for 3 min using a magnetic stirrer to prepare an emulsion by emulsification. Then 3, 5, 7 or 9 ml of ammonia water (ammonia concentration 28%) was added into the emulsion and stirred for 30 min continuously. Then a microsphere suspension was prepared by adding 40 ml of water and then it was filtered through a filter paper with the 11 mm pore size to collect microspheres. The obtained microspheres were dispersed into 80 ml of 0.1% polyvinyl alcohol again and stirred for 2 hours. After that, microspheres were obtained by filtering them through a filter paper and then dried for one night under the vacuum condition.

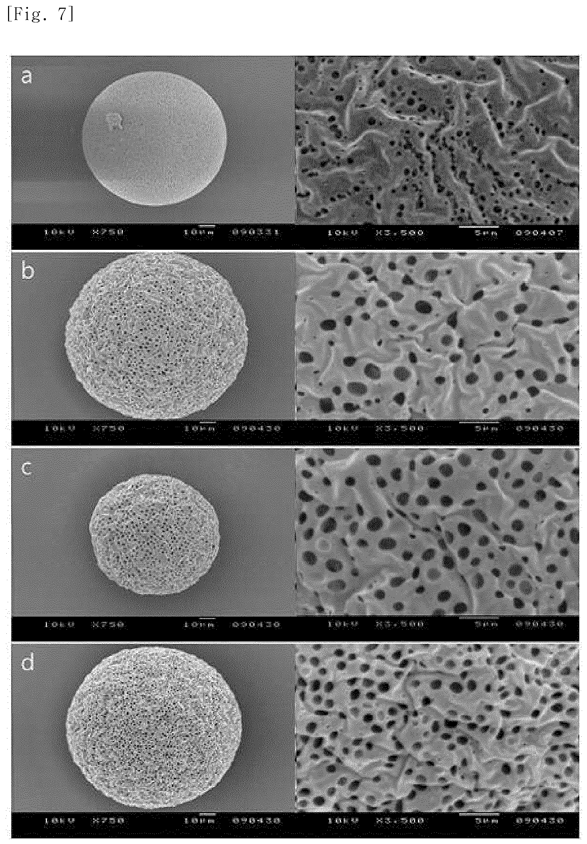

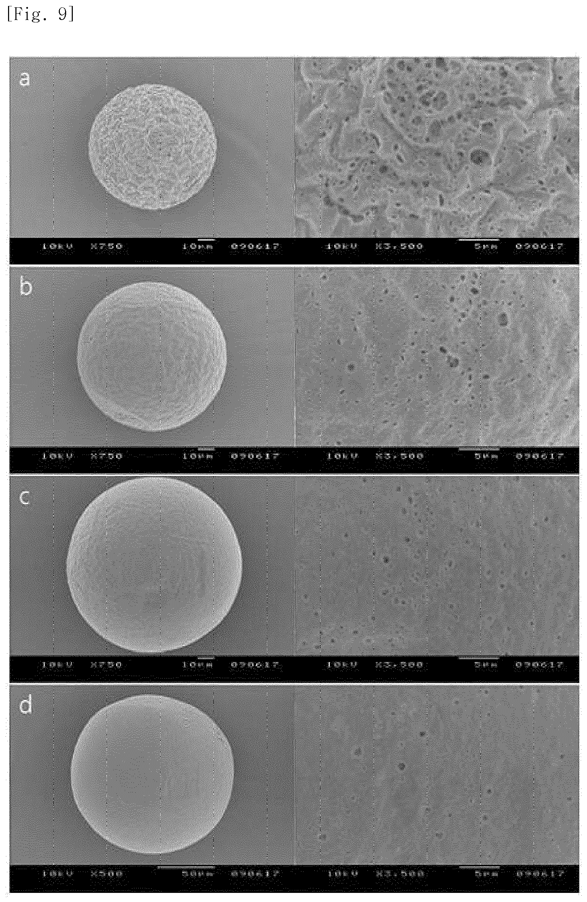

FIG. 7 showed that the porosity of microsphere surfaces and the number of air holes could be controlled according to the used amount of ammonia water which was added after o/w emulsion was prepared by fixing the used amount of PLGA and isopropyl formate. For example, when the capapcity of ammonia water added dropwise was increased from 3 ml to 9 ml, the porosity of microsphere surfaces were significantly increased. As shown in FIG. 9, the porosity of microsphere insides was also increased according to the used amount of ammonia water.

### Example 4. Preparation of microspheres

A dispersed phase was prepared by dissolving 0.15, 0.25, 0.35 or 0.45 g of PLGA (lactide:glycolide ratio = 65:35; inherent viscosity = 0.49 dL/g in chloroform) into 4 ml of isopropyl formate. The dispersed phase was dispersed into 40 ml of 0.5 %(w/v) polyvinyl alcohol (molecular weight = 25,000 g/mol; hydrolysis level = 88%) and stirred at 450 rpm for 3 min using a magnetic stirrer to prepare an emulsion by emulsification. Then 3 ml of ammonia water (ammonia concentration 28%) was added into the emulsion and stirred for 30 min continuously. Then a microsphere suspension was prepared by adding 40 ml of water and then it was filtered through a filter paper with the 11 mm pore size to collect microspheres. The obtained microspheres were dispersed into 80 ml of 0.1% polyvinyl alcohol again and stirred for 2 hours. After that, microspheres were obtained by filtering them through a filter paper and then dried for one night under the vacuum condition.

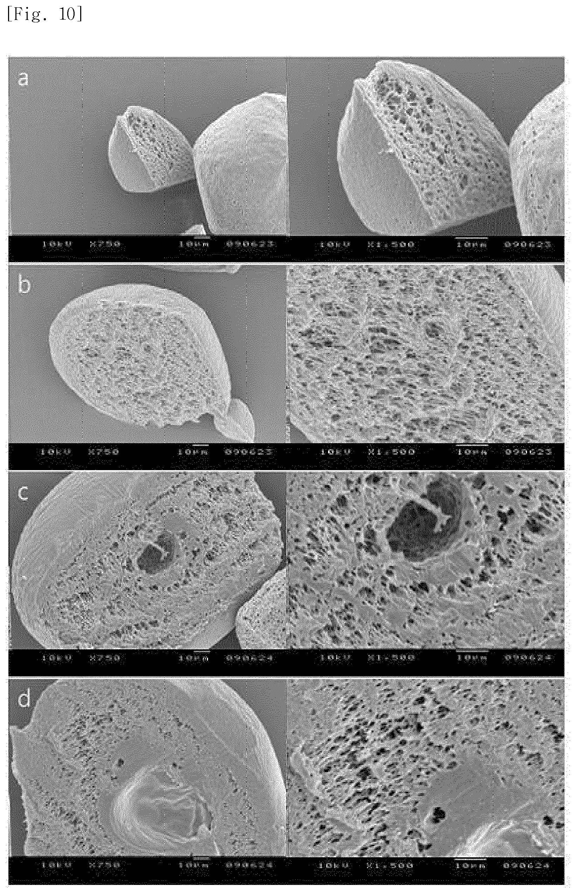

FIG. 9 is SEM photographs showing the surfaces of microspheres prepared respectively under the above conditions. When the capacity of LGA used for the preparation of microspheres was increased from 0.15 g to 0.25, 0.35 and 0.45, the phenomenon that the porosity of microsphere surfaces was gradually decreased. This tendency was same as the result shown in FIG. 3. However, microspheres shown in FIG. 3 were prepared by using PLGA materials of relatively low molecular weight, than microspheres shown in FIG. 9. In other words, it showed that the surface porosity of microspheres prepared according to the present invention could be controlled by not only the PLGA capacity composing the dispersed phase but also features like a molecular weight of PLGA materials. FIG. 10 shows internal SEM photographs of microspheres shown in FIG. 9. Since the surfaces of these microspheres were not easily peeled off by using a tape, insides of microspheres were exposed by being cut using a knife and observed. It was confirmed that the capacity of PLGA used to consist of the dispersed phase like as the surfaces of microspheres greatly affect the internal porosity of microspheres. Compared to the result shown in FIG. 4, it was demonstrated that features of PLGA itself (for example, viscosity/molecular weight) were also main factors determining the level of porosity of microsphere insides.

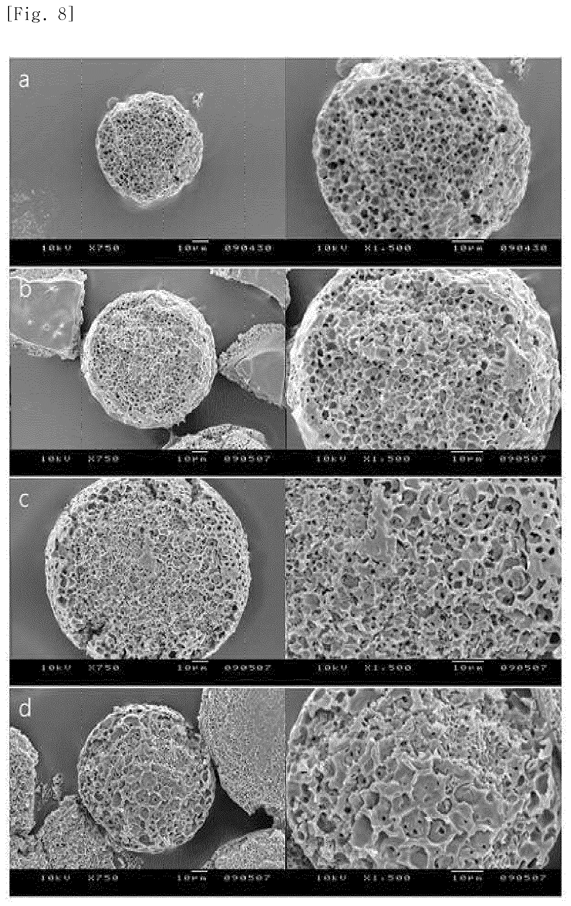

To compound the results shown in FIG. 1 to FIG. 10, the conclusion as follows could be drawn. It could be seen that the porosity and porous property were determined according to factors affecting the isopropyl formate decomposition reaction which occurred in the dispersed phase after forming emulsion and properties of dispersed phase. The first main factor is the used amount of PLGA. As long as the capacity of PLGA is increased under the condition in which the capacity of ammonia water and isopropyl formate is fixed, the viscosity of dispersed phase is increased. Accordingly, ammonolysis is relatively inhibited and the tendency that reaction degradation products are easily dispersed evenly is decreased. Therefore, PLGA-rich domains and PLGA-free domains were not easily separated in the dispersed phase, resulting in decreasing the porosity and the porous property. Likewise, when the molecular weight of used PLGA is increased, even though the used amount of isopropyl formate is fixed, the same effect as above is caused. Therefore, since when the low molecular weight of PLGA is used, than the case of using high molecular weight of PLGA, ammonolysis occurs well and degradation products are easily spread to the dispersed phase, thereby ultimately escaping into an aqueous phase, the porosity and porous property are increased. Secondly, as long as the used amount of isopropyl formate for preparing the dispersed phase as fixing the used capacity of PLGA and ammonia water is increased, the viscosity of prepared dispersed phase becomes decreased. This also facilitates the conditions of ammonolysis and at the same time, the ammonolysis products are easily spread into the dispersed phase and later escaped into the aqueous phase, thereby improving the porosity and porous property of microspheres. Likewise, it can be seen that when the used amount of isopropyl formate and the used amount of ammonia water were increased simultaneously after fixing the used amount of PLGA, according to aforementioned mechanism, the porosity and porous property of microspheres were improved. It is suggested that the porosity and porous property of microspheres could be controlled by controlling aforementioned various factors through the present invention.

### Example 5. Evaluation of spontaneous pore-closing functionality of porous microspheres

At first, 50 mg of microspheres prepared in the Example 3 (which was prepared by adding 3 ml of ammonia water after preparing the dispersed phase by dissolving 0.25 g of PLGA in 4 ml of isopropyl formate and emulsifying them in the aqueous phase) were dispersed into 30 ml of 0.5 % polyvinyl alcohol aqueous solution adjusted to 37 or 41.7. Microspheres were collected by using a filter paper per 0.5, 1, and 2 hours, softly stirring them using a magnetic stirrer, and then dried overnight under vacuum. The internal and external shapes of these microsphere samples were observed with SEM through the aforementioned methods.

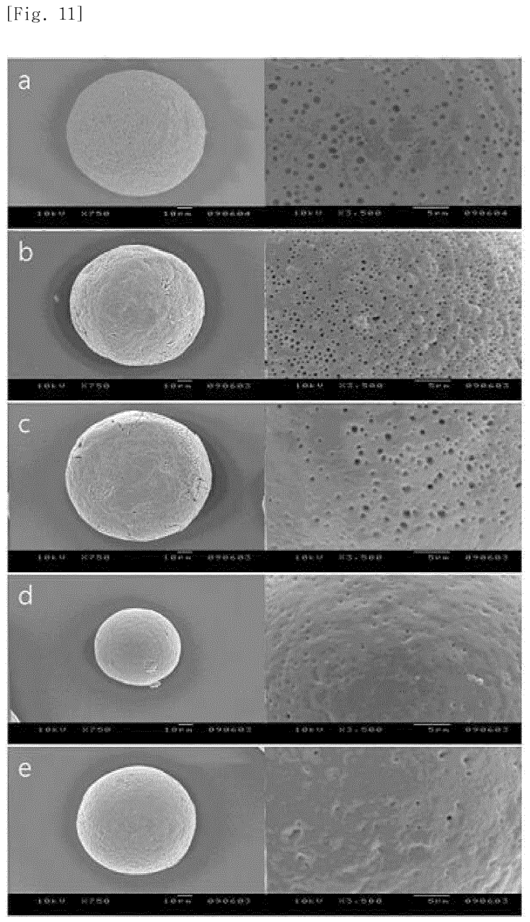

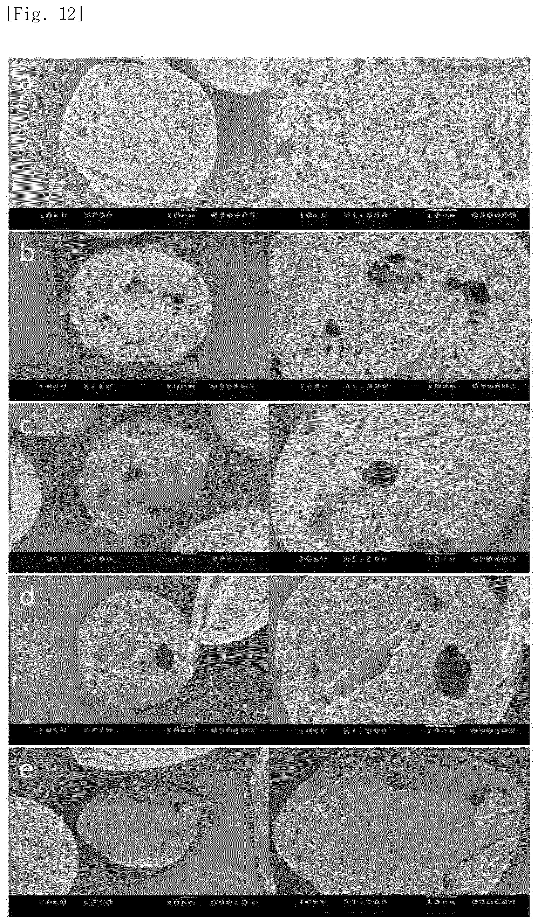

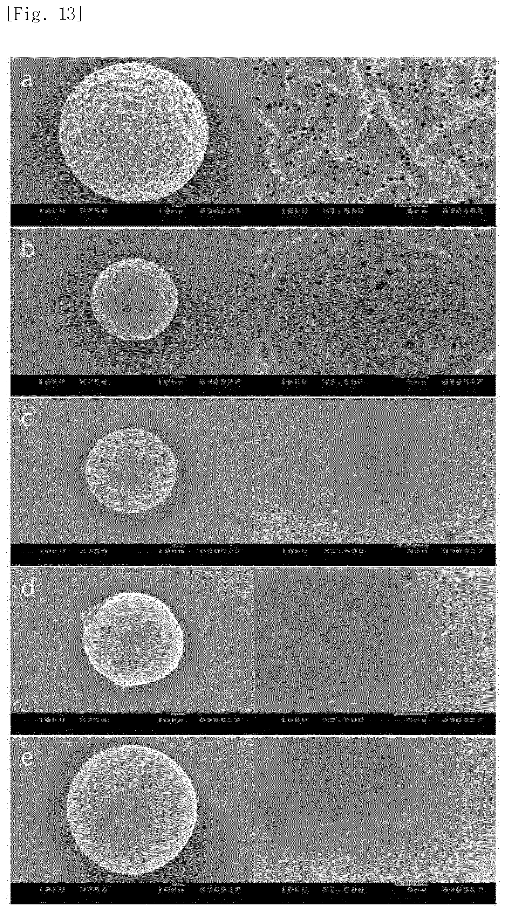

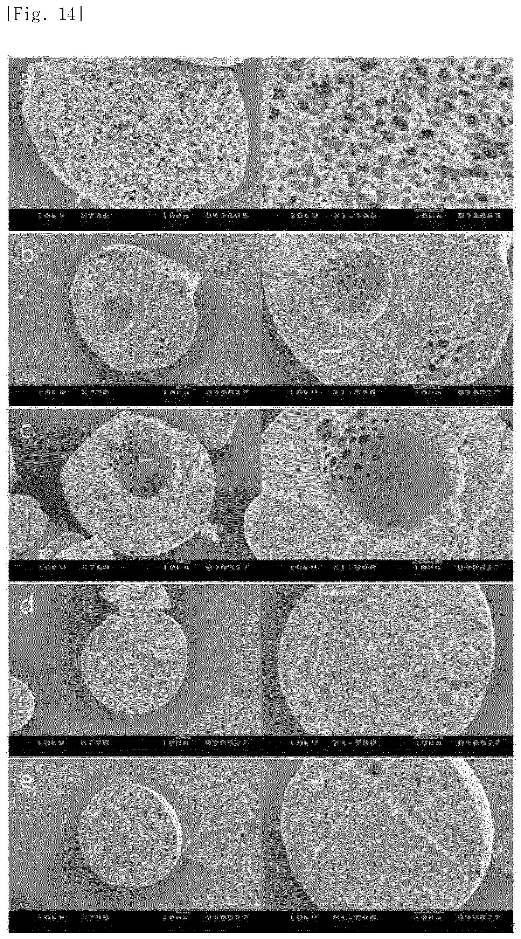

FIG. 11 showed the change of microsphere surfaces with time at 37 °C condition. The air holes present in the surfaces of microspheres exhibited the tendency of being closed with time. Numerous pores and air holes present in microsphere surfaces as well as microsphere insides also showed the tendency of being closed (FIG. 12). When the temperature of aqueous solution was increased to 41.7 °C, the rate of closing pores and air holes of surfaces and insides of microspheres became faster, thereby showing the functionality that internal and external pores and air holes were completely closed, when 2 hours passed (FIG. 13, FIG. 14). Porous PLGA microspheres in the sponge shape prepared in the present invention had the extremely strong porosity and the sponge shape in which numerous pores are connected to each other, with the differentiated preparation process, without using porogens. In addition, as shown in FIGs. 11, 12, 13 and 14, when these microspheres were dispersed in 100% aqueous solution which did not comprise an organic solvent, they had the functionality that pores present in surfaces and insides were slowly spontaneously closed. Moreover it was suggested that this spontaneous self-closing function could control its rate according to the temperature.

### Example 6. Confirmation of encapsulation aspect using FITC-dextran

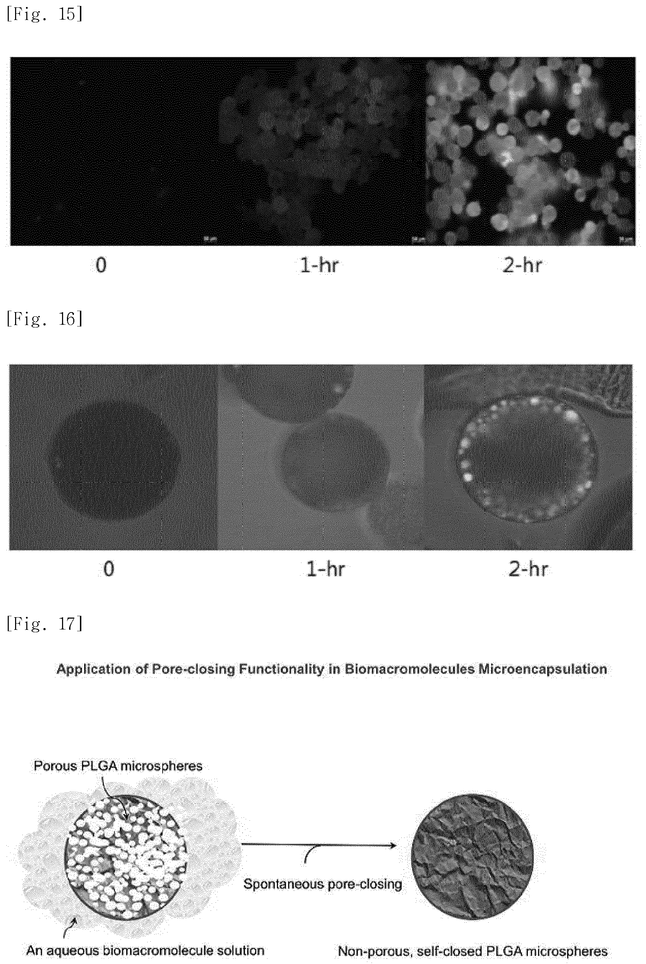

10 mg of microspheres prepared in the Example 3 (which was prepared by adding 7 ml of ammonia water after preparing the dispersed phase by dissolving 0.25 g of PLGA in 4 ml of isopropyl formate and emulsifying them in the aqueous phase) were dispersed into 100 ml of 0.2% Tween 80 aqueous solution in which 8 mg/ml of FITC-dextran (molecular weight = 40,000 g/mol) was included and left in 4 for one night. Then, after adjusting the temperature of microsphere suspension to 41, they were left for 2 hours. Afterward, after filtering and washing them with water, collected microspheres were observed by suing a fluorescence microscope and a confocal laser scanning microscope (model LSM 5140 Meta Confocal Laser Scanning Microscope, CLSM). At first, fluorescent images of samples were obtained by injecting 494 nm of light using Axiovert 200 revers fluorescence microscope (Carl Zeiss, Germany), thereby measuring 514 nm of fluorescence (FIG. 15). When 1 hour passed, microspheres exhibited fluorescence intensity, and when 2 hours passed, they exhibited much stronger fluorescence intensity. It was decided that it was not the fluorescence intensity according to the FITC-dextran which was not encapsulated in microspheres and existed in microsphere surfaces, because samples were sufficiently washed with water before the fluorescence microscope observation. That is, the result was shown according that pores of microspheres were spontaneously closed in the aqueous solution and the FITC-dextran dissolved in the aqueous solution was encapsulated into microspheres. Secondly, to obtain confocal laser scanning microscope images, 488 nm of argon laser light was injected into microsphere samples having spontaneous pore-closing function and 520 nm of fluorescence was collected through a release filter. As shown in FIG. 16, microspheres showed spontaneous pore-closing functionality according to the 41 °C exposure time of microsphere suspension passed, and it was demonstrated that the FITC-dextran was encapsulated through this process. This result showed that porous PLGA microspheres in the sponge shape having the spontaneous pore-closing function had the ability to encapsulate the wide range of active ingredients such as various drugs and biomolecules or solid nanoparticles (FIG. 17). In particular, without using an organic solvent and the like, under the condition of 100% aqueous phase, various kinds of active ingredients could be encapsulated into various sponge shape of PLGA microspheres prepared in advance.

### Example 7. Preparation of porous microspheres according to the present invention which uses ethyl cellulose as a polymeric compound

Ethyl cellulose of which viscosity was 10 cP in 5% toluene/ethanol was used.

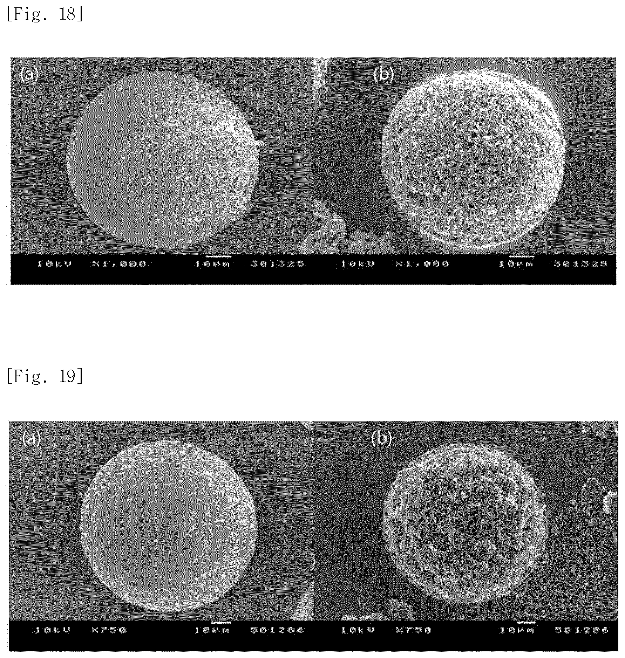

0.25 g of ethyl cellulose was dissolved in 4 ml of isopropyl formate using vortexing (Vortex GENIE 2, Scientific Industries, New York, USA) for 2 hours. This was added to a beaker in which 40 ml of 0.5% PVA solution was filled and stirred by using a stirrer (800 series, VWR scientific, Radnor, PA, USA) at 450 rpm for 3 min. Then 3 ml of 28% ammonia aqueous solution was added and stirred for 30 min, and filtered when 5 minutes were passed after adding 40 ml of distilled water. Microspheres left in the filter paper were re-dispersed in 80 ml of 0.1% PVA and stirred at 450 rpm for 2 hours, and then microspheres were collected through filtration and vacuum dried. The inside and outside shapes of microspheres were observed through SEM, and the result was shown in FIG. 18.

As can be seen in FIG. 18, even if microspheres according to the present invention were prepared by using ethyl cellulose as a polymeric compound, they had the extremely strong porosity and the sponge shape in which numerous pores were connected to each other.

### Example 8. Preparation of porous microspheres according to the present invention which uses polycaprolactone as a polymeric compound

Polycaprolactone was purchased from Sigma-Aldrich(Saint Louis, MO, USA), of which the average weight molecular weight (M_{w}) was 14,000 and the average quantity molecular weight(Mₙ) was 10,000.

0.25 g of polycaprolactone was dissolved in 4 ml of isopropyl formate using vortexing (Vortex GENIE 2, Scientific Industries, New York, USA) for 2 hours. This was added to a beaker in which 40 ml of 0.5% PVA solution was filled and stirred by using a stirrer (800 series, VWR scientific, Radnor, PA, USA) at 450 rpm for 3 min. Then 3 ml of 28% ammonia aqueous solution was added and stirred for 30 min, and filtered when 5 minutes were passed after adding 40 ml of distilled water. Microspheres left in the filter paper were re-dispersed in 80 ml of 0.1% PVA and stirred at 450 rpm for 2 hours, and then microspheres were collected through filtration and vacuum dried. The inside and outside shapes of microspheres were observed through SEM, and the result was shown in FIG. 19.

As can be seen in FIG. 19, even if microspheres according to the present invention were prepared by using polycaprolactone as a polymeric compound, they had the extremely strong porosity and the sponge shape in which numerous pores were connected to each other.

These results show that when the present invention is utilized, microspheres having the extremely strong porosity can be easily prepared, even though biodegradable PLGA-based polymers as well as different kinds of hydrophobic polymeric compounds forming microspheres are used.

## Claims

1. A method for preparing a polymeric microsphere in a sponge shape with spontaneous pore-closing functionality comprising
(a) a step of preparing a dispersed phase by contacting or mixing a polymeric compound having 1,000 to 80,000 of weight average molecular weight and an organic solvent;
(b) a step of preparation of emulsion which prepares oil-in-water (O/W) type of emulsion by adding a dispersion solvent into the dispersed phase prepared in the step (a); and
(c) a step of ammonolysis which performs ammonolysis by adding ammonia solution into the oil-in-water type of emulsion prepared in the step (b),
wherein the organic solvent is one or more non-halogen organic solvents selected from isopropyl formate, methyl formate, ethyl formate, propyl formate and mixtures of these organic solvents and a semipolar solvent.

2. The method for preparing a polymeric microsphere of claim 1, wherein the polymeric compound is one or more kinds selected from the group consisting of polylactic acid, polylactide, polylactic-co-glycolic acid, polylactide-co-glycolide (PLGA), polyphosphazene, polyiminocarbonate, polyphosphoester, polyanhydride, polyorthoester, copolymer of lactic acid and caprolacton, polycaprolacton, polyhydroxyvalerate, polyhydroxybutyrate, polyamino acid, cellulose esters, cellulose ethers, and copolymer of lactic acid and amino acid.

3. The method for preparing a polymeric microsphere of claim 1, wherein the semipolar solvent is one or more kinds selected from the group consisting of dimethyl sulfoxide, acetone, acetonitrile, tetrahydrofuran, dimethyl formamide and toluene.

4. The method for preparing a polymeric microsphere of claim 1, wherein the concentration of polymeric compound in the dispersed phase prepared in the step (a) is 3 to 30 (w/v)%.

5. The method for preparing a polymeric microsphere of claim 1, wherein the volume ratio of dispersed phase : dispersion solvent is 1:1 to 1:100 in the step (b).

6. The method for preparing a polymeric microsphere of claim 1, wherein the dispersion solvent used in the step (b) is an aqueous dispersion solvent comprising an emulsifier.

7. The method for preparing a polymeric microsphere of claim 1, wherein the dispersion solvent used in the step (b) is an aqueous solution comprising polyvinyl alcohol as an emulsifier.

8. The method for preparing a polymeric microsphere of claim 1, wherein the mole ration of organic solvent: ammonia solution is 1:0.5 to 1:20.

9. A polymeric microsphere with spontaneous pore-closing functionality prepared by the method of any one of claims 1 to 8, having pores, wherein the pores have a sponge shape of an open structure interconnected within the microsphere.

10. A composition for bioactive substance delivery comprising the polymeric microsphere according to any one of claims 1 to 8 and a bioactive substance, wherein the bioactive substance is encapsulated in the polymeric microsphere.

11. The composition for bioactive substance delivery of claim 10, wherein the bioactive substance is a biomacromolecule.

12. The composition for bioactive substance delivery of claim 11, wherein the biomacromolecule is peptide, polypeptide, polynucleotide, protein, DNA, RNA, nanoparticle, monoclonal antibody or vaccine.

13. A method for preparing a composition for bioactive substance delivery comprising a step of preparing the spontaneous pore-closing polymeric microsphere according to any one of claims 1 to 8; and
a step of encapsulating a bioactive substance into the microsphere.

14. A method for delivery of a bioactive substance comprising a step of administering the composition for bioactive substance delivery according to any one of claims 10 to 12.
